# EUROPEAN PATENT SPECIFICATION

(11) **EP 2 450 077 B1**
(45) Date of publication and mention of the grant of the patent: **24.10.2018**
(21) Application number: 10793559.5
(22) Date of filing: 17.06.2010
(51) Int. Cl.: A61M 25/00, A61B 17/00

(54) **MICRO CATHETER**
MIKROKATHETER
MICROCATHÉTER

(30) Priority: 30.06.2009 CN 200910054209
(43) Date of publication of application: 09.05.2012
(73) Proprietor: MicroPort NeuroTech (Shanghai) Co., Ltd., Shanghai 201318 (CN)
(72) Inventor: JIN, Qiaorong, Shanghai 201203 (CN); XIE, Zhiyong, Shanghai 201203 (CN); LI, Yu, Shanghai 201203 (CN); WANG, Sen, Shanghai 201203 (CN); XIANG, Yonggang, Shanghai 201203 (CN); LUO, Qiyi, Shanghai 201203 (CN)
(74) Representative: Dennemeyer & Associates S.A.
(86) International application number: PCT/CN2010/073997
(87) International publication number: WO 2011/000264

(56) References cited:
- EP-B1- 0 643 979
- WO-A1-2008/130740
- WO-A1-2010/021908
- WO-A2-02/05885
- CN-Y- 2 889 337
- JP-A- 2005 312 952
- JP-A- 2006 181 258
- JP-A- 2007 229 245
- JP-A- 2007 319 594
- JP-A- 2008 036 157
- US-A- 5 630 806
- US-A1- 2004 079 429

## Description

The present application claims a priority of the Chinese patent application with the application No. **"**200910054209-5**",** entitled "**Micro Catheter**", which was submitted with the Chinese Patent Office on June 30, 2009.

### Technical Field

The present invention relates to a catheter, and, in particular, to a medical micro catheter for treating lesions in the blood vessel by a nerve intervention method.

### Background Art

The interventional treatment refers to a minimally invasive endovascular operative treatment by cutting a small hole in a certain position of the human body, and then inserting a micro catheter into a blood vessel in the body of a patient to perform repairing, expanding, and dredging works using high-tech devices such as a television monitor. The interventional treatment as an effective treatment method for various blood vessel diseases has already gained global attention.

A catheter body, which is the main body of the micro catheter, is required to meet very high demands, i.e., it needs to have sufficient flexibility, torque transmission, follow-up characteristics, supporting property, kink resistance, etc., so as to ensure that the micro catheter can be bent along the blood vessel of the patient and respond to the operations such as pushing-pulling and twisting made by the doctor duly and exactly during the operation. In addition, in order to deliver medication, guide wires or an embolism substance such as a stent, a coil, etc., to the affected part along the catheter, the entire length of the catheter body must also have a hollow cavity. Thus, the catheter body of the micro catheter has a very special and complicated structure.

However, the micro catheter used in a traditional interventional treatment is generally only adapted to a specific blood vessel part, e.g., the coronary artery, and cannot be adapted to other blood vessel parts, e.g., intracranial distal blood vessels. Even if the size of the micro catheter is reduced to be smaller than the size of the intracranial distal blood vessel in proportion, the scaled down micro catheter still cannot be adapted to the intracranial distal blood vessel. This is also easily understood, for these traditional micro catheters are just designed for blood vessels such as the coronary artery, and the sizes and bending radiuses thereof are both greatly larger than those of the intracranial distal blood vessels. With regard to the intracranial distal blood vessels, since they are more complicated and tortuous, they require higher flexibility, torque transmission, follow-up characteristics, supporting property, and kink resistance of the catheter body. Thus, in order to be used for the intracranial distal blood vessels, the traditional micro catheter should be significantly changed in structure.

In addition, several micro catheters for intracranial nerve intervention have been announced. These new catheter structures are completely different in structure and design from the traditional micro catheters. However, the outer diameters of the distal ends of these catheters can be only reduced to about 0.8 mm, and the properties of these catheters cannot completely meet the requirements for nerve intervention.

WO 2008/130740 discloses a catheter having a wall portion including a polymeric bonding layer and a reinforcing layer. The reinforcing layer is embedded between an inner layer and an outer layer and comprises a braided portion and a coiled portion, whereby the coiled portion is distal to the braided portion.

WO 02/05885 discloses a medical device with a tube for performing invasive procedures. The tube comprises a polymeric bonding layer and an inner liner. The polymeric layer includes the reinforcing layer made of a braid and a coil, whereby the braid extends at least partly over the coil.

### Summary of the Invention

The object of the present invention is to provide a catheter with improved flexibility, torque transmission, follow-up characteristics, supporting property, kink resistance, and the ability to be adaptable to the distal blood vessels of the brain.

This object is solved by a catheter having the features of claim 1.

Though different from the existing several catheters, the catheter in the present invention does not employ a new structure completely different from the catheter body of the traditional micro catheter, but makes improvements in terms of structure on several aspects based on the catheter body of the traditional micro catheter adapted to the coronary artery, etc. This catheter not only can be reduced in size, but also have notably improved flexibility, torque transmission, follow-up characteristics, supporting property, and kink resistance, and thus can pass through the intracranial distal blood vessels safely and favorably.

According to one aspect of the present invention, a catheter is put forward. A catheter wall of the catheter comprises a smooth layer, a reinforcing layer and an outer covering layer in the order from inside to outside, wherein the smooth layer and the outer covering layer are both made of polymer materials, and the reinforcing layer is a supporting layer, which is connected with the smooth layer and the outer covering layer respectively, so as to connect the respective layers of the catheter together. In addition, the catheter comprises a proximal portion and an extension portion in the direction from the proximal end to the distal end, wherein the reinforcing layer assumes a braid structure at the proximal portion, and assumes a helix structure at the extension portion. By means of this design, the flexibility, torque transmission, follow-up characteristics, supporting property, and kink resistance of the catheter are all notably improved, and a balance between the pushing property at the proximal end and the flexibility at the distal end is achieved. Preferably, the catheter is a single-lumen catheter.

Further, the braid structure of the reinforcing layer of the catheter at the proximal end assumes a diamond network structure, and preferably, the number of pitch points per inch (PPI) of the diamond braid structure increases in the direction from the proximal end to the distal end, and especially continuously increases from 50 PPI to 180 PPI. By means of this design, the catheter is made to have a better supporting property, flexibility and force uniform transmissibility.

Further, an included angle α between a helix winding wire of the helix structure at the extension portion of the reinforcing layer of the catheter and the axial direction is in a range of 45-90°. Preferably, the included angle α changes from 50°to 85°.

Further, a pitch of the helix structure continuously decreases in the direction from the proximal end to the distal end, and preferably continuously decreases from 0.5 mm to 0.1 mm. By means of this design, the catheter is made to have a better supporting property, flexibility and force uniform transmissibility.

Further, the extension portion comprises a transition portion and a distal portion, wherein the outer diameter of the distal portion continuously decreases from 1.2 mm to 0.5 mm in the direction from the proximal end to the distal end.

According to another aspect of the present invention, a micro catheter for nerve intervention is put forward. The micro catheter comprises a connector, a diffusion-induced stress tube and a catheter body that are connected in order, and further comprises a radiopaque member of a helix shape at the distal portion of the catheter body. Besides, the connector, the diffusion-induced stress tube and the catheter body all comprise a tubular lumen, wherein the proximal end of the diffusion-induced stress tube is inserted into the lumen of the connector, and the proximal end of the catheter body is inserted into the lumen of the diffusion-induced stress tube. And the catheter body of the micro catheter comprises the above-mentioned catheter.

### Brief Description of the Drawings

The embodiments of the present invention are described below by referring to the figures only in a manner of giving examples:
Fig. 1 is a schematic diagram that shows the entire structure of the micro catheter in a preferred embodiment of the present invention;
Fig. 2 is a schematic diagram that shows the structure of the reinforcing layer of the micro catheter in a preferred embodiment of the present invention;
Fig. 3 is a partial enlarged view of the reinforcing layer shown in Fig. 2, which shows the changing situation of the number of pitch points and the pitch of the reinforcing layer of the micro catheter;
Fig. 4A shows the diamond structure of the reinforcing layer at the proximal portion shown in Fig. 2;
Fig. 4B shows the helix winding structure of the reinforcing layer at the extension portion shown in Fig. 2;
Figs. 5A-5C are views of the cross section of the micro catheter in preferred embodiments of the present invention, wherein Fig. 5A and Fig. 5B respectively show two structure solutions of the layered catheter body at the proximal portion, and Fig. 5C shows the structure solution of the layered catheter body at the extension portion;
Figs. 6A-6C are sectional views of the outer covering layer of the micro catheter along the axis in preferred embodiments of the present invention, which respectively show optional structure solutions of the outer covering layer; and
Fig. 7 is a schematic diagram that shows the hardness change of the outer covering layer of the micro catheter in a preferred embodiment of the present invention.

### Detailed Description

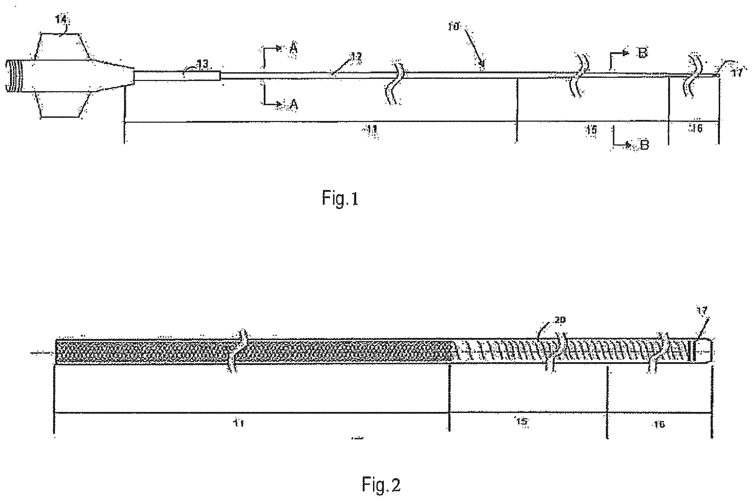

Fig. 1 is a schematic diagram that shows the entire structure of the micro catheter in a preferred embodiment of the present invention. As shown in Fig. 1, the micro catheter 10 mainly comprises a connector 14, a diffusion-induced stress tube 13 and a catheter body 12 that are connected in the order from the proximal end to the distal end. In order to better facilitate the descriptions, throughout the present patent, the direction close to the operator during the operation is called "proximal", and the direction away from the operator during the operation is called "distal".

The connector 14 and the diffusion-induced stress tube 13 both employ a well-known structure, and have hollow tubular lumens, the inner diameters of which are respectively substantially the same as the outer diameters of the proximal ends of the diffusion-induced stress tube 13 and the catheter body 12. The proximal end of the diffusion-induced stress tube 13 is inserted from the distal end of the lumen of the connector 14, and these two parts are adhered together by means of glue. The proximal end of the catheter body 12 is also inserted from the distal end of the lumen of the diffusion-induced stress tube 13, and passes through the entire diffusion-induced stress tube 13 to enter the lumen of the connector 14, and the catheter body 12 and the diffusion-induced stress tube 13 are also adhered together by means of glue. The catheter body 12 also has a hollow tubular lumen, which, however, is not used for connection, but is used for delivering medication, guide wires or an embolism substance such as a stent, a coil, etc.

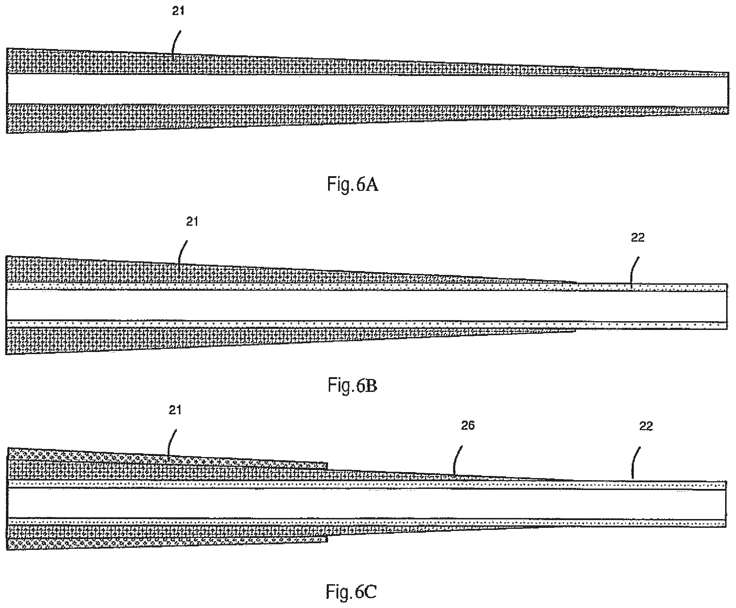

The outer diameter of the entire catheter body 12 gradually decreases in a certain gradient from the proximal end to the distal end (as shown in Fig. 1 and Figs. 6A-6C), which gives the catheter body 12 a very good crossing property. The outer diameter of the distal portion 16 continuously decreases from 1.2 mm to 0.5 mm from the proximal end to the distal end, which is sufficient to enter the distal blood vessels of the human brain. In contrast, the outer diameter of the micro catheter for the coronary artery is about 1.5 mm, which is much larger than that of the micro catheter according to the present invention. The minimum outer diameter of the distal end of the micro catheter for nerve intervention, which is totally different from the traditional micro catheter, of the other existing structures can only come up to 0.8 mm.

The catheter body 12 can be divided into a proximal portion 11, a transition portion 15 and a distal portion 16 in the order from the proximal end to the distal end, wherein the length of the proximal portion 11 is 125 cm, the length of the transition portion 15 is 25 cm, and the length of the distal end 16 is 5 cm.

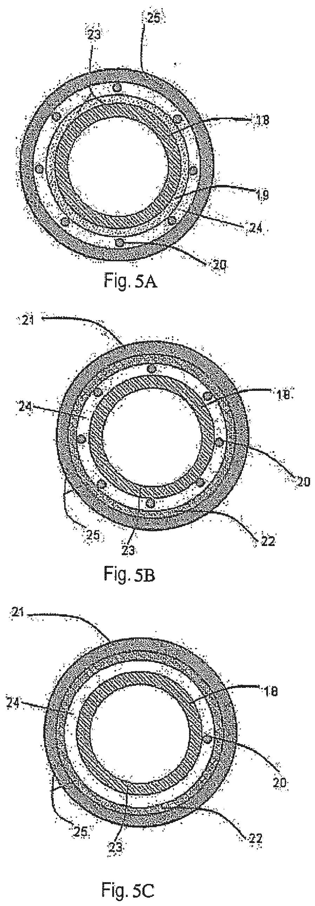

The internal structure of the catheter body 12 will be described in detail below by referring to Figs. 5A-5C. As shown in Figs. 5A-5C, the catheter body 12 in the present embodiment is a single-cavity catheter body that assumes a multi-layered structure, which comprises a smooth layer 23, a reinforcing layer 24 and an outer covering layer 25 in the order from inside to outside in the radial direction. The smooth layer 23 and the outer covering layer 25 both employ the prior art.

The smooth layer 23 can be a single-layered structure (as shown in Figs. 5B and 5C) or a multi-layered structure (as shown in Fig. 5A). The innermost layer 18 of the single-layered structure or multi-layered structure of the smooth layer 23 is a smooth tube made of polymer materials of a low friction coefficient such as polytetrafluoroethylene or high-density polyethylene so as to facilitate pass of the guide wires, etc., and the other layers of the multi-layered structure are all supporting layers 19 of the smooth layer 23, and are made of a well-known material such as polyimide, etc.

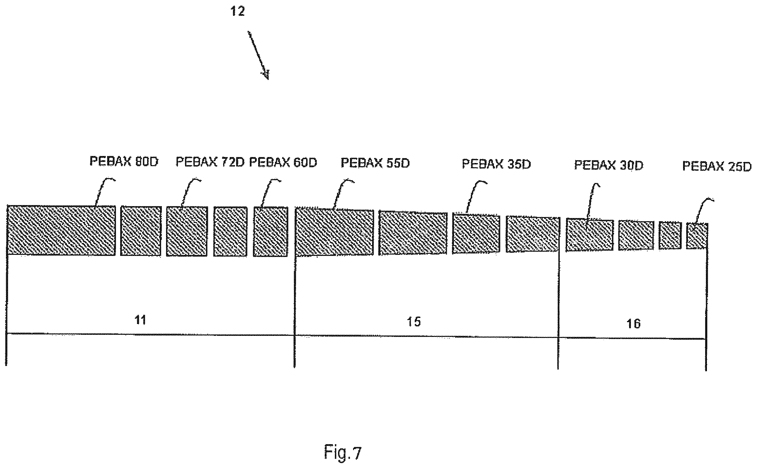

The outer covering layer 25 continuously covers each of the regions of the reinforcing layer 24. The material for the outer covering layer is generally one or more of the thermoplastic polymer materials such as polyamide, polyurethane or polyether, e.g., PU, PEBAX, PE, NYLON, PA, etc. In order to improve the pushing property of the catheter body 12, the polymer material for the outer covering layer 25 can be extruded or adhered in accordance with a gradually changing hardness gradient. As shown in Fig. 7, the hardness of the outer covering layer 25 (or the outermost layer) of the proximal portion 11 gradually changes from 80D to 60D from the proximal end to the distal end, the hardness of the outer covering layer 25 of the transition portion 15 gradually changes from 55D to 35D from the proximal end to the distal end, and the hardness of the outer covering layer 25 of the distal portion 16 gradually changes from 30D to 25D from the proximal end to the distal end.

Similar to the smooth layer 23, the outer covering layer 25 can be also a single-layered structure (as shown in Fig. 5A) or a multi-layered structure (as shown in Figs. 5B and 5C and Figs. 6B and 6C). When the outer covering layer 25 is a multi-layered structure, the outer covering layer 25 comprises an innermost layer material 22 and an outermost layer material 21, and several intermediate layers 26 can be further included between the above two layers (as shown in Fig. 6C), wherein the innermost layer material 22 generally covers the entire region of the reinforcing layer 24, and the hardness of the innermost layer material 22 is comparatively small relative to the other layers, so that the distal portion has a comparatively good flexibility. The material for the intermediate layers 26 is generally harder than the innermost layer material 22, so that the force can be uniformly transferred during the pushing process of the catheter body 12, and the wall thickness of the intermediate layers 26 gradually decreases from the proximal end to the distal end. In addition, the intermediate layers 26 do not need to cover the whole innermost layer material 22, but can cover a part of the innermost layer material 22 close to the proximal end. The hardness of the outermost layer material 21 is generally higher than those of the other layers, so that the catheter body 12 has a good supporting property to thereby avoid the occurrence of a bending or knotting phenomenon during the process of pushing the catheter. When there are no intermediate layers (as shown in Fig. 6B), the outermost layer material 21 can directly cover the innermost layer material 22, and when there are intermediate layers (as shown in Fig. 6C), the outermost layer material 21 covers the intermediate layers 26. In addition, the outermost layer material 21 generally only covers the proximal portion of the innermost layer material 22 or the intermediate layers 26.

The reinforcing layer 24 is located between the smooth layer 23 and the outer covering layer 25, and the design of the reinforcing layer 24 serves an important function with respect to all the properties of the catheter body 12. In order to achieve a good pushing property for adaptation to tortuous and complicated cerebral vessel paths, requirements for the properties of the proximal portion 11, the transition portion 15 and the distal portion 16 of the catheter body 12 are different, wherein the proximal portion 11 is required to have a better supporting property, the transition portion 15 is required to have a better force uniform transmissibility, and the distal portion 15 is required to have a better flexibility.

To meet the above requirements, in the present embodiment, the reinforcing layer 24 assumes a braid structure at the proximal portion 11, and assumes a helix structure at the transition portion 15 and the distal portion 16.

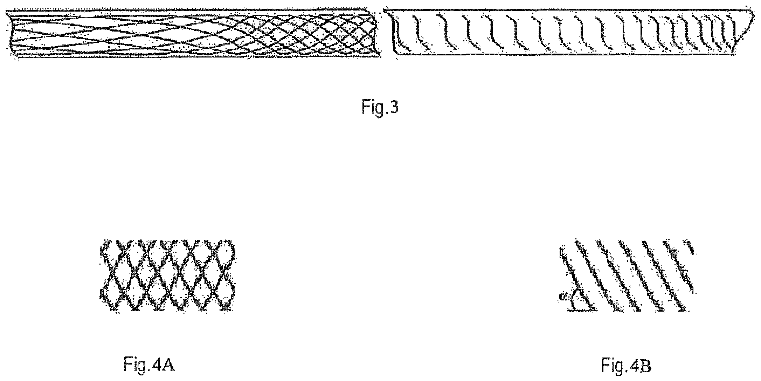

To be specific, the reinforcing layer 24 of the proximal portion 11 is formed into a diamond structure by dozens of metal wires (e.g., medial stainless steel wires or nickel-titanium alloy wires) by a traditional weaving method (as shown in Figs. 2, 3 and 4A), so that the proximal portion 11 of the catheter body 12 not only has a good supporting property but also has a good torque transmission. In order to further improve the property of the reinforcing layer 24, a gradually changing number of braid pitch points of the reinforcing layer can be employed (as shown in Fig. 3) to thereby improve the supporting property, flexibility and force uniform transmissibility from the proximal end to the distal end in the axial direction of the catheter body 12. For example, in the present embodiment, the braid pitch of the reinforcing layer 24 of the proximal portion 11 gradually changes from 50 PPI at the proximal-most end to 180 PPI.

Furthermore, at the transition portion 15 and the distal potion 16, the reinforcing layer 24 is formed by helix winding of one metal wire (as shown in Fig. 2 and Fig. 3) to thereby effectively ensure the flexibility of the transition portion 15 and the distal portion 16. The kink resistance and the compression resistance of this part of catheter body 12 are closely associated with the included angle α between the helix winding wire 20 of the transition portion 15 and the distal portion 16 and the axial direction. For example, in the present embodiment, α is 45°, but can be also an arbitrary angle in the range of 45-90°, and certainly can be a changing angle, e.g., an angle gradually changing from 50°to 85°. In addition, in order to improve the supporting property, flexibility and force uniform transmissibility from the proximal end to the distal end in the axial direction of the catheter body 12, the helix winding wire can also employ a gradually changing pitch, e.g., a pitch gradually decreasing from 0.5 mm at the proximal-most end of the transition portion 15 to 0.1 mm at the distal-most end of the distal portion 16 in the present embodiment.

The above contents only relate to the preferred embodiments of the present invention, and it should be noted that those skilled in the art can also make several improvements, decorations or changes in case of not breaking away from the principle of the present invention, e.g., the cutting ring can be a part of the corresponding pipeline or an independently provided component having a comparatively high wear resistance. These improvements, decorations or changes should also be deemed as ones within the scope of protection of the present invention.

## Claims

1. A catheter, a catheter wall of which comprises an inner smooth layer (23) made of a material having a low friction coefficient so as to facilitate pass of guidewires, a reinforcing layer (24) and an outer covering layer (25) in the order from inside to outside, the inner layer (23) and the outer covering layer (25) being both made of polymer materials, the reinforcing layer (24) being a supporting layer, which is connected with the inner layer (23) and the outer covering layer (25) respectively, so as to connect the respective layers of the catheter together; and
the catheter comprising a proximal portion (11) and an extension portion in the direction from the proximal end to the distal end, whereby
the reinforcing layer (24) assumes a braid structure at the proximal portion (11), and assumes a helix structure at the extension portion,
**characterized in that** an included angle α between a helix winding wire (20) of the helix structure and the axial direction is in a range of 45-90° and that a pitch of the helix structure continuously decreases in the direction from the proximal end to the distal end.

2. The catheter according to claim 1, **characterized in that** the braid structure assumes a diamond shape.

3. The catheter according to claim 2, **characterized in that** the number of pitch points of the diamond braid structure continuously increases in the direction from the proximal end to the distal end.

4. The catheter according to claim 3, **characterized in that** the number of pitch points changes in a range of 50-180 PPI.

5. The catheter according to claim 4, **characterized in that** the number of pitch points continuously increases from 50 PPI to 180 PPI.

6. The catheter according to claim 1, **characterized in that** the included angle α gradually changes from 50° to 85° .

7. The catheter according to claim 1, **characterized in that** the pitch of the helix structure changes in a range of 0.1-0.5 mm.

8. The catheter according to claim 7, **characterized in that** the pitch of the helix structure continuously decreases from 0.5 mm to 0.1 mm.

9. The catheter according to any one of claims 1-8, **characterized in that** the hardness of the outer covering layer (25) continuously decreases in the direction from the proximal end to the distal end.

10. The catheter according to any one of claims 1-8, **characterized in that** the outer diameter of the catheter continuously decreases in the direction from the proximal end to the distal end.

11. The catheter according to claim 10, **characterized in that** the extension portion (15, 16) comprises a transition portion (15) and a distal portion (16), and the outer diameter of the distal portion (16) changes in a range of 0.5-1.2 mm.

12. The catheter according to claim 11, **characterized in that** the outer diameter of the distal portion (16) continuously decreases from 1.2 mm to 0.5 mm.

13. The catheter according to any one of claims 1-10, **characterized in that** the material for the reinforcing layer (24) is a stainless steel or a nickel-titanium alloy.

14. A micro catheter for nerve intervention, **characterized in that** a catheter body (12) of the micro catheter comprises the catheter according to claims 1-13.

## Patentansprüche

1. Katheter, dessen eine Katheterwand Folgendes umfasst: eine innere glatte Schicht (23) aus einem Material mit einem niedrigen Reibungskoeffizienten, um den Durchgang von Führungsdrähten zu erleichtern, eine Verstärkungsschicht (24) und eine äußere Deckschicht (25) in dieser Reihenfolge von innen nach außen, wobei die innere Schicht (23) und die äußere Deckschicht (25) beide aus Polymermaterialien bestehen, wobei die Verstärkungsschicht (24) eine Trägerschicht ist, die jeweils mit der inneren Schicht (23) und der äußeren Deckschicht (25) verbunden ist, um die jeweiligen Schichten des Katheters miteinander zu verbinden; und wobei der Katheter einen proximalen Abschnitt (11) und einen Verlängerungsabschnitt in der Richtung vom proximalen zum distalen Ende umfasst, wodurch
die Verstärkungsschicht (24) am proximalen Abschnitt (11) eine Geflechtstruktur aufweist und am Verlängerungsabschnitt eine Wendelstruktur aufweist,
**dadurch gekennzeichnet, dass** ein eingeschlossener Winkel a zwischen einem Wendelwicklungsdraht (20) der Wendelstruktur und der axialen Richtung in einem Bereich von 45 bis 90° liegt und dass eine Steigung der Wendelstruktur in der Richtung vom proximalen zum distalen Ende kontinuierlich abnimmt.

2. Katheter nach Anspruch 1, **dadurch gekennzeichnet, dass** die Geflechtstruktur eine Rautenform aufweist.

3. Katheter nach Anspruch 2, **dadurch gekennzeichnet, dass** die Anzahl der Wälzpunkte der Rautengeflechtstruktur in der Richtung vom proximalen zum distalen Ende kontinuierlich zunimmt.

4. Katheter nach Anspruch 3, **dadurch gekennzeichnet, dass** sich die Anzahl der Wälzpunkte in einem Bereich von 50 bis 180 PPI ändert.

5. Katheter nach Anspruch 4, **dadurch gekennzeichnet, dass** die Anzahl der Wälzpunkte kontinuierlich von 50 PPI auf 180 PPI zunimmt.

6. Katheter nach Anspruch 1, **dadurch gekennzeichnet, dass** sich der eingeschlossene Winkel a graduell von 50° auf 85° ändert.

7. Katheter nach Anspruch 1, **dadurch gekennzeichnet, dass** sich die Steigung der Wendelstruktur in einem Bereich von 0,1 bis 0,5 mm ändert.

8. Katheter nach Anspruch 7, **dadurch gekennzeichnet, dass die** Steigung der Wendelstruktur kontinuierlich von 0,5 mm auf 0,1 mm abnimmt.

9. Katheter nach einem der Ansprüche 1 bis 8, **dadurch gekennzeichnet, dass** die Härte der äußeren Deckschicht (25) in der Richtung vom proximalen zum distalen Ende kontinuierlich abnimmt.

10. Katheter nach einem der Ansprüche 1 bis 8, **dadurch gekennzeichnet, dass** der Außendurchmesser des Katheters in der Richtung vom proximalen zum distalen Ende kontinuierlich abnimmt.

11. Katheter nach Anspruch 10, **dadurch gekennzeichnet, dass** der Verlängerungsabschnitt (15, 16) einen Übergangsabschnitt (15) und einen distalen Abschnitt (16) aufweist und der Außendurchmesser des distalen Abschnitts (16) sich in einem Bereich von 0,5 bis 1,2 mm ändert.

12. Katheter nach Anspruch 11, **dadurch gekennzeichnet, dass** der Außendurchmesser des distalen Abschnitts (16) kontinuierlich von 1,2 mm auf 0,5 mm abnimmt.

13. Katheter nach einem der Ansprüche 1 bis 10, **dadurch gekennzeichnet, dass** das Material für die Verstärkungsschicht (24) ein Edelstahl oder eine Nickel-Titan-Legierung ist.

14. Mikrokatheter zum Nerveneingriff, **dadurch gekennzeichnet, dass** ein Katheterkörper (12) des Mikrokatheters den Katheter nach den Ansprüchen 1 bis 13 umfasst.

## Revendications

1. Cathéter, dont une paroi de cathéter comprend une couche lisse interne (23) constituée d'un matériau présentant un faible coefficient de frottement afin de faciliter le passage de fils de guidage, une couche de renforcement (24) et une couche de recouvrement externe (25) dans l'ordre de l'intérieur vers l'extérieur, la couche interne (23) et la couche de recouvrement externe (25) étant toutes deux constituées de matériaux polymères, la couche de renforcement (24) étant une couche de support reliée à la couche interne (23) et à la couche de recouvrement externe (25) respectivement, de manière à relier ensemble les couches respectives du cathéter ; et
le cathéter comprenant une partie proximale (11) et une partie d'extension dans la direction de l'extrémité proximale à l'extrémité distale, grâce à quoi
la couche de renforcement (24) adopte une structure de tresse au niveau de la partie proximale (11) et adopte une structure en hélice au niveau de la partie d'extension,
**caractérisé en ce que** l'angle inclus a entre un fil d'enroulement en hélice (20) de la structure en hélice et la direction axiale est compris dans une plage de 45 à 90° et qu'un pas de la structure en hélice diminue continuellement dans la direction de l'extrémité proximale à l'extrémité distale.

2. Cathéter selon la revendication 1, **caractérisé en ce que** la structure de tresse adopte la forme d'un diamant.

3. Cathéter selon la revendication 2, **caractérisé en ce que** le nombre de points primitifs de la structure de tresse en forme de diamant augmente continuellement dans la direction de l'extrémité proximale à l'extrémité distale.

4. Cathéter selon la revendication 3, **caractérisé en ce que** le nombre de points primitifs varie dans une plage de 50 à 180 PPI.

5. Cathéter selon la revendication 4, **caractérisé en ce que** le nombre de points primitifs augmente continuellement de 50 à 180 PPI.

6. Cathéter selon la revendication 1, **caractérisé en ce que** l'angle inclus a varie progressivement de 50° à 85°.

7. Cathéter selon la revendication 1, **caractérisé en ce que** le pas de la structure en hélice varie dans une plage de 0,1 à 0,5 mm.

8. Cathéter selon la revendication 7, **caractérisé en ce que** le pas de la structure en hélice diminue continuellement de 0,5 mm à 0,1 mm.

9. Cathéter selon l'une quelconque des revendications 1 à 8, **caractérisé en ce que** la dureté de la couche de recouvrement externe (25) diminue continuellement dans la direction de l'extrémité proximale à l'extrémité distale.

10. Cathéter selon l'une quelconque des revendications 1 à 8, **caractérisé en ce que** le diamètre extérieur du cathéter diminue continuellement dans la direction de l'extrémité proximale à l'extrémité distale.

11. Cathéter selon la revendication 10, **caractérisé en ce que** la partie d'extension (15, 16) comprend une partie de transition (15) et une partie distale (16), et le diamètre extérieur de la partie distale (16) varie dans une plage de 0,5 à 1,2 mm.

12. Cathéter selon la revendication 11, **caractérisé en ce que** le diamètre extérieur de la partie distale (16) diminue continuellement de 1,2 mm à 0,5 mm.

13. Cathéter selon l'une quelconque des revendications 1 à 10, **caractérisé en ce que** le matériau pour la couche de renforcement (24) est un acier inoxydable ou un alliage de nickel-titane.

14. Microcathéter pour une intervention sur un nerf, **caractérisé en ce qu'**un corps de cathéter (12) du microcathéter comprend le cathéter selon les revendications 1 à 13.
